(19) Europäisches Patentamt
European Patent Office
Office européen des brevets

(11) **EP 2 783 714 B1**

(12) **EUROPÄISCHE PATENTSCHRIFT**

(45) Veröffentlichungstag und Bekanntmachung des Hinweises auf die Patenterteilung:
**18.05.2016  Patentblatt 2016/20**

(51) Int Cl.:
*A61M 1/36* (2006.01)    *A61M 1/16* (2006.01)

(21) Anmeldenummer: **14158083.7**

(22) Anmeldetag: **06.03.2014**

(54) **Verfahren und Vorrichtung zur Bestimmung eines Rezirkulationszustands**

Method and device for determining a recirculation state

Procédé et dispositif de détermination d'un état de recirculation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2013  DE 102013103220**

(43) Veröffentlichungstag der Anmeldung:
**01.10.2014  Patentblatt 2014/40**

(73) Patentinhaber: **B. Braun Avitum AG**
**34212 Melsungen (DE)**

(72) Erfinder: **Wolff, Henrik**
**37213 Witzenhausen (DE)**

(74) Vertreter: **Winter, Brandl, Fürniss, Hübner, Röss, Kaiser, Polte - Partnerschaft mbB**
**Patent- und Rechtsanwaltskanzlei**
**Bavariaring 10**
**80336 München (DE)**

(56) Entgegenhaltungen:
**EP-A1- 0 900 094**        **EP-A1- 1 083 948**
**WO-A1-95/32010**        **WO-A1-2011/026647**
**WO-A1-2012/062257**        **DE-A1-102010 015 664**
**US-A1- 2002 062 098**

## Beschreibung

Die vorliegende Erfindung betrifft ein Verfahren und eine Vorrichtung zur dialysemaschinenseitigen Bestimmung oder Messung (Erkennung) einer Rezirkulation innerhalb eines patientenseitigen Ein-/Auslass-Elements (Patientenzugang), vorzugsweise eines Shunts.

## Hintergrund der Erfindung

Die Dialyse ist ein extrakorporales Blutreinigungsverfahren, das bei Nierenversagen als Ersatzverfahren zum Einsatz kommt. Die Dialyse ist neben der Nierentransplantation eine der effizientesten und damit wichtigsten Nierenersatztherapien bei chronischem Nierenversagen und eine der Behandlungsmöglichkeiten bei akutem Nierenversagen. Unter Dialyse bzw. allgemein Blutreinigung dieser Gattung wird dabei ein Stoffaustausch über eine Membran verstanden, wobei auf der einen Membranseite Blut/Plasma und auf der anderen Seite der Membran eine Dialysierflüssigkeit anliegt oder entlang strömt.

Während einer Behandlung wird hierfür über einen Patientenzugang (Shunt) Blut aus dem Patienten gepumpt, im Dialysator (Filter) an einer Dialysemembran vorbeigeführt und in gereinigtem Zustand dem Patienten wieder zurückgegeben. Giftstoffe (Stoffwechselabbauprodukte) bzw. urämische Toxine aus klein- und mittelmolekulare Substanzen (membrangängige Stoffe) werden in einem Hämodialyse-, Hämofiltrations- oder Hämodiaflitrations-Betriebsmodus (-Behandlungsmodus) der Dialysemaschine aus dem Blut hauptsächlich durch Diffusion und/oder Konvektion über die Membran auf die andere Filterseite in die Dialysierflüssigkeitslösung gefördert und dadurch entfernt. Der Dialysator wird mit Ausnahme bei der Hämofiltration ständig von frischer Dialysierflüssigkeit durchströmt (vorzugsweise ca. 500 - 800 ml/min), wobei die Strömungsrichtung der Dialysierflüssigkeit vorzugsweise in Gegenstromrichtung zur Blutströmung in den Dialysator eingeleitet wird.

Eine Hämodialysebehandlung wird in der Regel ca. 4 - 5 Stunden (Nachtdialyse bis 8 Stunden) pro Behandlung und mindestens dreimal in der Woche durchgeführt (abhängig von Körpergewicht, Nierenrestfunktion, Herzleistung). Patienten, die Heim - Hämodialyse durchführen, vermeiden das problematische längere Behandlungsintervall am Wochenende und dialysieren häufiger, im Regelfall alle zwei Tage oder sogar täglich.

Ein entscheidender Einflussfaktor auf die Qualität der Dialyse ist der Blutfluss. Je höher dieser ist, desto besser ist im Allgemeinen das Behandlungsergebnis. Allerdings kann es vorkommen, dass im patientenimplantierten Dialyseshunt zur Blutentnahme und Rückführung des gereinigten Blutes Probleme auftreten (z.B. eingeschränkte bzw. nicht mehr ausreichende Durchströmung infolge falscher/mangelnder Positionierung im Patienten, Beschädigung, etc.), insbesondere wenn der Blutfluss im extrakorporalen System hoch ist. Die genaue Höhe des Blutflusses, bei dem diese Probleme auftreten, hängt von der Qualität des Patientenzugangs und dem Blutfluss durch den Shunt sowie dessen Unversehrtheit (keine Stenosen oder Aneurismen) ab.

Sowohl bei einem geringen Blutfluss durch den Shunt, als auch in dem Zustand, in welchem der Shuntfluss noch deutlich höher ist als der eingestellte Blutfluss, kann das Problem/Phänomen einer sogenannten "Rezirkulation" im Shunt auftreten. Das bedeutet, dass schon gereinigtes Blut aus der am Patienten angelegten venösen Nadel wieder von der ebenfalls am Patienten angelegten arteriellen Nadel angesaugt wird und sich mit dem noch zu reinigenden Blut vermischt. Somit wird die Reinigungsleistung geschwächt und möglicherweise kein zufriedenstellendes Behandlungsresultat erzielt. Gleiches kann auch geschehen, wenn beim Anlegen des Patienten an die Dialysemaschine die arterielle und venöse Nadel fälschlicher Weise vertauscht werden. Dann befindet sich die venöse Nadel stromaufwärts der arteriellen Nadel und es tritt ebenfalls ein Rezirkulationseffekt auf. Fluidische Effekte führen analog zu Rezirkulation bei geringer Shuntqualität.

## Stand der Technik

Zurzeit erfolgt die Rezirkulationsmessung über komplexe Verfahren, die beispielsweise auf einer Bolus-Gabe beruhen (beispielsweise NaCl oder Temperatur oder Leitfähigkeit).

Ein grundsätzliches Ziel der meisten dieser Verfahren (wie auch der vorliegenden Erfindung) ist es, ein System zu etablieren, das über eine möglichst einfache maschineninterne Messung insbesondere zu Beginn einer Behandlung die Rezirkulation bestimmen kann.

Die Bestimmung von Rezirkulation im Shunt des Patienten wurde bereits im Stand der Technik durch verschiedene Verfahren vorgestellt. So wird bei an sich bekannten Dialysegeräten die Erkennung der Rezirkulation beispielsweise durch eine Temperaturkontrolle (EP 0 900 094) realisiert. Dazu wird das extrakorporal geführte Blut im venösen Leitungsabschnitt mit einem Bolus in Form eines Temperaturpulses versehen. Die Temperaturmessung im arteriellen Leitungssystem ermöglicht dabei den Rückschluss auf den prozentualen Anteil der Rezirkulation.

Die WO 2011/026647 A1 offenbart eine Vorrichtung zur Bestimmung von entsprechenden Konzentrations- bzw. Absorbanzwerte in Dialysierflüssigkeit, ohne die Therapie- bzw. Behandlungszeit des Patienten zu verlängern, so das nicht nur ökonomischer therapiert werden kann, sonder auch eine zusätzliche Belastung des Patienten durch eine länger andauernde Therapie bzw. Behandlung minimiert wird. Dabei soll auch hinsichtlich der Messeinrichtung für die Absorbanz der apparative Aufwand minimal gehalten werden. Die Rezirkulation ist in dieser Offenbarung ein Kriterium durch das die Dialyse negativ beeinflusst wird. Eine etwaige Bestimmung der Rezirkulation wird in dieser Schrift nicht durchgeführt

Die WO 2012/062257 A1 offenbart eine Methode und eine Vorrichtung zur Bestimmung von entfernten harnpflichtigen Substanzen aus einem extrakorporalen Blutkreislauf und einer anhand dieser Daten gesteuerten Flussanpassung. Es wird ein online-Monitoring-System zur Verfügung gestellt, mit dessen Hilfe der Fluss der Dialyselösung und/oder des Blutes gesteuert werden kann. Die Konzentrationsbestimmung der harnpflichtigen Substanzen wird über eine photometrische Messung (UV) durchgeführt. Die über ein photometrisches Konzentrationsmessystem gewonnen Daten können nicht nur zur Analyse und zur Erfolgskontrolle des Dialysevorgangs verwendet, sondern auch zur Berechnung der Regelung der Blutbehandlungseinheit genutzt werden.

Des Weiteren wird im einschlägigen Stand der Technik eine Messung nach Art einer Off-line Dialyse angeboten, welches auf Basis einer Leitfähigkeitsmessung erfolgt und ebenfalls mit der Gabe eines geeignetes Bolus arbeitet. Hierbei wird die Rezirkulation im Shunt eines Dialysepatienten durch die Messung der Leitfähigkeit ermittelt. Auch bei diesem Verfahren wird im venösen Schlauchabschnitt ein Elektrolytbolus verabreicht, welcher die Leitfähigkeit der Flüssigkeit modifiziert. Durch die Bestimmung der Leitfähigkeit im arteriellen Schlauchabschnitt kann somit auf den Grad der Rezirkulation zurückgeschlossen werden (US 7,815,852).

Darüber hinaus kann Rezirkulation prinzipiell auch mit Hilfe eines Hämatokrit - Sensors bestimmt werden. Dabei wird der Hämatokrit des Blutes durch die Gabe eines definierten Bolus im venösen Leitungsabschnitt geändert. Durch die Bestimmung des Hämatokrits im arteriellen Leitungssystem ist die Bestimmung der Rezirkulation möglich. Technische Vorschläge, die auf diesem Prinzip basieren, sind beispielsweise in der US 5,685,989 offenbart. Diese bekannten Produkte beruhen entweder auf der Messung der Rezirkulation mittels elektromagnetischer Strahlung im sichtbaren Bereich, oder auf der Messung der Rezirkulation durch Ultraschall.

## Probleme des Stands der Technik

Die beschriebenen Verfahren/Produkte können die Rezirkulation im Patientenzugang auf unterschiedlichen Wegen bestimmen. Allerdings ist bei allen vorstehend genannten Verfahren die Gabe eines Bolus im venösen bzw. arteriellen Abschnitt des extrakorporalen Leitungssystems nötig, welcher anschließend im arteriellen Abschnitt gemessen wird.

Darüber hinaus ist es erforderlich, dass der verabreichte Bolus definierte Eigenschaften besitzt, welche sich von dem extrakorporalen Blut deutlich unterscheiden, sodass dieser messtechnisch zweifelsfrei detektierbar ist. Auch sind je nach Verfahren ein bis mehrere zusätzliche Detektoren/Sensoren notwendig, um den Bolus quantitativ und qualitativ auswerten zu können. Die besondere Schwierigkeit bei diesen Verfahren ist jedoch die Tatsache, dass die extrakorporalen Leitungssysteme in ihrer Qualität und Beschaffenheit stark voneinander abweichen, was bei den Messverfahren nicht berücksichtigt werden kann. Dieser Umstand führt u.U. dazu, dass Messergebnisse verfälscht werden.

## Kurzbeschreibung der Erfindung

Die Aufgabe der Erfindung ist es daher, ein Steuerungsverfahren und eine Vorrichtung zur Bestimmung der Rezirkulation im Shunt bereit zu stellen, das/die ohne eine Boluszugabe auskommt und daher nicht die Blut- oder Dialysierflüssigkeitsseite durch einen Bolus (Störung) beeinflusst.

Ein Ziel ist es, dass für die Durchführung einer Rezirkulationsmessung gemäß der Erfindung keine weiteren (Mess-) Geräte auf der Blutseite benötigt werden, da nur Maschinenparameter steuerungstechnisch variiert werden sollen, um eine Messung (vorzugsweise im on-line Modus) zu ermöglichen. Des Weiteren soll die Messung ausschließlich auf der Dialysierflüssigkeitsseite - und damit ohne zusätzlichen technischen Aufwand auf der Blutseite - des Gerätes erfolgen.

Diese Aufgabe wird durch ein Steuerungsverfahren sowie eine Vorrichtung mit den Merkmalen der Ansprüche 1 und 10 gelöst. Vorteilhafte Ausgestaltungen und/oder Weiterbildungen der Erfindung sind Gegenstand der Unteransprüche.

Ein Grundgedanke der vorliegenden Erfindung besteht darin, den Blutfluss ausgehend von einem Referenzwert (z.B. ein Blutfluss von 50 ml/min), bei welchem wissentlich / nachweislich der Zustand einer Komplettreinigung (und fehlender / vernachlässigbarer Rezirkulation) vorliegt, auf einen gewünschten individuellen Behandlungswert (z.B. einen Soll - Blutfluss von durchschnittlich 300 ml/min) einzustellen / zu verändern. Während dieser Einstellung / Veränderung wird die Systemantwort, d.h. die Systemreaktion bezüglich einer aktuell messbaren Ist - Kenngröße (repräsentiert direkt oder indirekt die Konzentration an urämischen Toxinen im gewünschten Behandlungsbetriebsmodus von beispielsweise 300 ml/min), ausgelöst durch die Blutflussänderung, mit Hilfe eines Sensors ermittelt und über eine rechnergestützte Vergleichseinheit mit einer aus einer zuvor gemessenen Referenz - Kenngröße berechneten Soll - Kenngröße ins Verhältnis gesetzt, wobei so aus der gemessenen Ist - Kenngröße und der errechneten Soll - Kenngröße ein Ist - Vergleichswert berechnet wird, der schließlich mit einem systemspezifischen (im Vorfeld ermittelten und hinterlegten) Soll - Vergleichswert verglichen und hieraus ein Differenzwert ermittelt werden kann. Dieser Differenzwert steht in Korrelation zu einem zugehörigen Rezirkulationsgrad für den eingestellten Blutfluss (gewünschter Behandlungsbetriebsmodus).

Dieser Rezirkulationsgrad ist dadurch gekennzeichnet, dass schon gereinigtes Blut über mindestens ein Bluteinlass-/Auslasselement (können auch zwei baulich/räumlich getrennte Elemente sein) unabhängig von dessen konstruktiver Ausgestaltung und/oder Anordnung am/im Patientenkörper in den extrakorporalen Kreislauf ein- und ausgeleitet wird, wodurch sich der extrakorporale Kontaminationsgrad gegenüber dem intrakorporalen Kontaminationsgrad ändert. Die Rezirkulation kann dabei verursacht werden durch:

- die Konstruktion/Anordnung des mindestens einen Einlass-/Auslasselements, (auch bekannt unter Shuntrezirkulation) oder

- die Zirkulation durch das kardiopulmonale System (auch bekannt unter kardiopulmonale Rezirkulation).

An dieser Stelle sei darauf hingewiesen, dass unter dem Begriff "ohne/fehlende Rezirkulation" ein Zustand verstanden werden soll, bei welchem eine Rezirkulation zwischen 0 und einem vernachlässigbaren Wert vorherrscht. Beispielsweise kann bei vertauschter arterieller und venöser Nadel auch bei einem Referenzblutfluss von 50ml/min Rezirkulation auftreten. Allerdings wäre dies bei typischen Herzzeitvolumina von beispielsweise 4000 ml/min vernachlässigbar klein (50/4000) und hätte keine Auswirkungen auf das erfindungsgemäße Verfahren. Des Weiteren soll unter dem Begriff "Komplettreinigung" der folgende Zustand verstanden werden:

Ein Komplettreinigung einer Substanz aus dem Patientenblut liegt definitionsgemäß dann vor, wenn die Konzentration der jeweiligen Substanz im venösen (rückführenden) Schtauchsegment "Null" ist.

Im Nachfolgenden wird aus Vereinfachungsgründen für die aus dem Dialysator abströmende, verbrauchte Dialysierflüssigkeit auch der Begriff "Dialysierflüssigkeits - Kontamination" verwendet.

Die vorstehend erläuterte Vorgehensweise findet ihre Anwendung für den Fall, dass das Blutreinigungssystem beispielsweise in einem Hämodialysemodus betrieben wird, also in einem Betriebsmodus, in welchem eine unverbrauchte Dialysierflüssigkeit mit einem vorbestimmten Volumenstrom > 0 durch einen Dialysator geführt wird. Es ist aber alternativ gemäß einem anderen Grundgedanken der vorliegenden Erfindung auch möglich, das System in einem Filtrationsmodus (reine Konvektion) zu betreiben, bei dem keine neue / frische Dialysierflüssigkeit dem Dialysator zugeführt wird, also der Stofftransport von der Blutseite des Dialysators über die Membran in die Dialysierflüssigkeitskammer gelangt, wobei auch in diesem Fall beispielsweise für einen Blutfluss von 50ml/min als Referenzwert keine Rezirkulation vorliegt. Die dabei ermittelte Referenz - Kenngröße braucht nun nicht mehr in eine (theoretische) Soll - Kenngröße für den beabsichtigten Behandlungsbetriebsmodus (z.B. 300ml/min) hochgerechnet werden (d.h. eine Clearancebestimmung ist nicht notwendig), sondern kann für diesen speziellen Ultrafiltrationsmodus unmittelbar mit einer aktuell messbaren Ist - Kenngröße im gewünschten Behandlungsbetriebsmodus (z.B. 300ml/min) ins Verhältnis gesetzt werden.

Im Konkreten ausgedrückt, es wird grundsätzlich (lediglich) ein Sensor zum Bestimmen einer optischen Absorbanz / Absorbtion (Referenz - Kenngröße und Ist - Kenngröße) auf der Dialysierflüssigkeitsseite (also nicht Blutseite) einer Blutreinigungsmaschine eingesetzt, welcher vorzugsweise mit elektromagnetischer Strahlung im UV-Bereich arbeitet. Dabei soll ausdrücklich darauf hingewiesen werden, dass auch andere online - Messverfahren bekannter Art einsetzbar sind. Ein solches UV-Messverfahren der bevorzugten Art ist beispielsweise in der Arbeit von Fridolin et al (Uhlin F., Haemodialysis treatment monitored online by ultraviolet absorbance, Linköping University Medical Dissertations no. 962, Department of Medicine and Care Division of Nursing Science & Department of Biomedical Engineering, 2006) und ein entsprechender Sensor in der Patentschrift EP 1 083 948 beschrieben, sodass an dieser Stelle auf diesen allgemein bekannten und somit zum Fachwissen zählenden Stand der Technik verwiesen werden kann.

Konkrete Grundlage für das erfindungsgemäße Messverfahren in einem ersten bevorzugten Fall eines Hämodialysemodus (HD-Modus) als Betriebsmodus ist es, dass bei einem bekannten Blutfluss von im bevorzugten Ausführungsbeispiel 50 ml/min (d.h. vorbekannter Referenzblutfluss für Referenzwertbestimmung) eine Komplettreinigung bei fehlender Rezirkulation erreicht wird (beispielsweise vollständige Befreiung des Bluts von urämischen Toxinen im Fall eines UV - Messverfahrens). In diesem Fall kann erfindungsgemäß ausgehend von einem Absorbanzwert als bevorzugte gemessene / messbare Ist - Kenngröße für den jeweiligen / aktuellen (Behandlungs-) Blutfluss unter Miteinbeziehung eines zuvor gemessenen Referenz - Absorbanzwerts (bevorzugte Referenz - Kenngröße) die Ist - Clearance als ein bevorzugter Ist - Vergleichswert (Anteil des Blutflusses zum Gesamtblutfluss, der vollständig von urämischen Toxinen gereinigt ist) bei jedem beliebigen aktuellen (Behandlungs-) Blutfluss einfach berechnet werden. Auf der Basis der Clearance - Bestimmung lässt sich schließlich der Rezirkulationsgrad für den angestrebten Behandlungsblutfluss herleiten. Hierfür können die folgenden steuerungstechnischen Verfahrensschritte sequenziell ausgeführt werden:

a. Bestimmung / Messung einer Referenz - Kenngröße, beispielsweise des (Referenz-) Absorbanzwerts A(50) in der verbrauchten (aus dem Dialysator abströmenden) Dialysierflüssigkeit bei einem Referenz - Blutfluss vorzugsweise von 50 ml/min (unter der Voraussetzung der Komplettreinigung und fehlender Rezirkulation),

b. Bestimmung / Messung einer Ist - Kenngröße, beispielsweise des Ist - Absorbanzwertes in der verbrauchten Dialysierflüssigkeit bei dem gewählten / gewünschten (Soll -) Behandlungsfluss A(BBF) (vorzugsweise bei 300 ml/min) als Systemreaktion,

c. Aus der Referenz - Kenngröße, vorzugsweise dem (Referenz -) Absorbanzwert A(50) bei 50 ml/min kann eine Soll - Kenngröße, vorzugsweise der theoretische (Soll -) Wert A(BBF,theo) der Absorbanz bei angenommener (theoretischer) Komplettreinigung und (theoretischer) Rezirkulationsfreiheit des gewählten Behandlungsflusses A(BBF) (vorzugsweise 300 ml/min) wie folgt errechnet werden: A(50) * (BBFml/min/50ml/min)=A(BBF,theo)

d. Aus dem Verhältnis der gemessenen Ist - Kenngröße, vorzugsweise des Ist - Absorbanzwerts A(BBF) (bei tatsächlich vorliegender, ggf. unvollständiger Reinigung) zu der theoretisch errechneten/hochgerechneten Soll - Kenngröße, vorzugsweise dem Soll - Absorbanzwert A(BBF, theo) (bei theoretischer Komplettreinigung) lässt sich ein Ist - Vergleichswert, vorzugsweise die aktuelle Clearance K(BBF, ist) (angegeben in ml/min) bestimmen, indem man dieses Verhältnis mit dem Blutfluss BBF, multipliziert, nämlich: K=[A(BBF) / A(BBF,theo)] * BBF

e. Anschließend kann der ermittelte Ist - Vergleichswert, vorzugsweise die aktuelle Clearance K(BBF, ist) mit einem vorbestimmten (filterspezifischen) sowie hinterlegten Soll - Vergleichswert, beispielsweise eine Soll - Clearance K(BBF, theo) für diesen Filter bei ausbleibender Rezirkulation verglichen werden. Dieser Wert wird für jeden Filter individuell ermittelt und beispielsweise auf einer Speichereinheit hinterlegt oder manuell eingegeben.

Aus der durch den Vergleich gemäß Verfahrensschritt e. ermittelten Abweichung, vorzugsweise Clearanceabweichung $\Delta$LK (d.h. ($K_{theo}$ - $K_{ist}$)) lässt sich abschließend die Rezirkulation Rez errechnen bzw. bestimmen/definieren.

Alternativ hierzu kann als Grundlage für das erfindungsgemäße Messverfahren das System gemäß einem zweiten bevorzugten Fall in einem Ultrafiltrationsmodus (reine Filtration) als Betriebsmodus gefahren werden. Hierbei wird ebenfalls eine Referenz - Kenngröße bei einem Referenzblutfluss gemessen, bei dem bekanntermaßen keine Rezirkulation auftritt, beispielsweise bei einem Blutfluss von 50 ml/min. Da hierbei ein Schadstoffübergang aus dem Blut in eine Dialysierflüssigkeit ausschließlich durch Konvektion (Dialysierflüssigkeits - Volumenstrom = 0) am Dialysator stattfindet, ist eine Clearancebestimmung in diesem Fall nicht notwendig. D.h., jetzt wird die Referenz - Kenngröße unmittelbar mit der Ist - Kenngröße für den gewünschten Behandlungsblutfluss (z.B. 300 ml/min) ins Verhältnis gesetzt, woraus sich dann die Rezirkulation im gewünschten Behandlungs - Betriebsmodus herleiten lässt. Hierfür können die folgenden Verfahrensschritte sequenziell ausgeführt werden:

a. Bestimmung / Messung einer Referenz - Kenngröße, beispielsweise der (Referenz-) Absorbtion/Absorbanz bei einem Referenz - Blutfluss vorzugsweise von 50 ml/min (unter der Voraussetzung fehlender Rezirkulation),

b. Bestimmung / Messung einer Ist - Kenngröße, beispielsweise der Ist - Absorbtion / Ist - Absorbanz bei dem gewählten / gewünschten (Soll -) Behandlungsfluss BBF (vorzugsweise bei 300 ml/min) als Systemreaktion,

c. Aus der gemessenen Referenz - Kenngröße, vorzugsweise der (Referenz -) Absorbtion bei 50 ml/min kann eine weitere Kenngröße beispielsweise die Referenz - Absorbanz A(50) bestimmt werden, die mit einer Soll - Kenngröße, vorzugsweise dem theoretischen (Soll -) Wert A(BBF,theo) der Absorbanz für eine angenommene (theoretische) Rezirkulationsfreiheit beim gewählten Behandlungsfluss BBF (vorzugsweise 300 ml/min) als identischer Wert gleichgesetzt werden kann: A(50) = A(BBF,theo)

d. Aus dem Verhältnis der gemessenen Ist - Kenngröße, vorzugsweise des Ist - Absorbanzwerts A(BBF) (bei tatsächlich vorliegender, ggf. unvollständiger Reinigung) zu der Soll - Kenngröße, vorzugsweise dem Soll - Absorbanzwert A(BBF, theo) (bei theoretischer Komplettreinigung ohne Rezirkulation) der hier dem Referenz - Wert A(50) entspricht, lässt sich umittelbar die Rezirkulation Rez ermitteln aus:

$$Rez\ (in\ \%) = (1 - (A(BBF) / A(50))) * 100\ oder$$

$$Rez\ (in\ ml/min) = (1 - (A(BBF) / A(50))) * BF$$

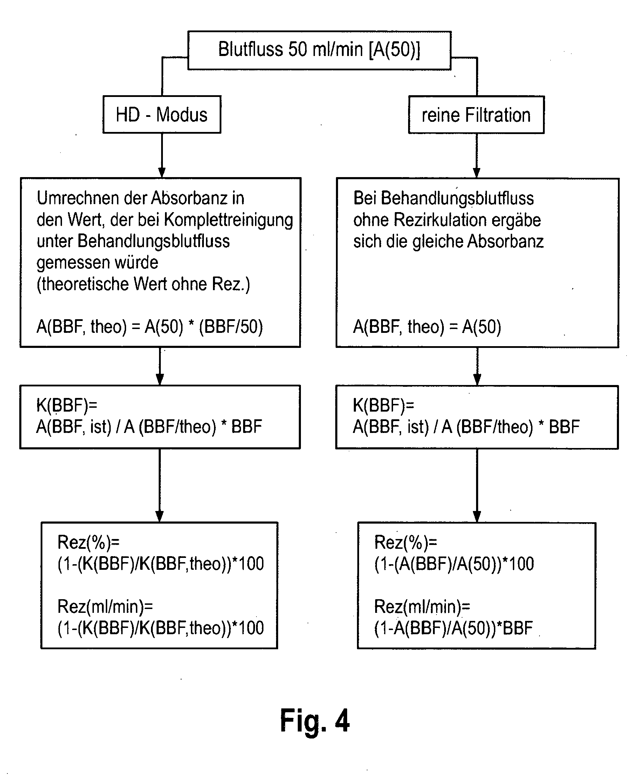

Diese beiden alternativen Steuerungsverfahren, welche dem gleichen Verfahrenprinzip folgen und nur an unterschiedliche Betriebsmodi, nämlich dem Hämodialysemodus und dem Hämofiltrationsmodus angepasst sind, werden in der anliegenden Fig. 4 in Gegenüberstellung dargestellt. Dieses, aus der Fig. 4 entnehmbare grundlegende Verfahrensprinzip oder Steuergerüst lässt sich in Kenntnis der beiden vorstehenden alternativen Erfindungsgedanken wie Folgt umschreiben:

- Bestimmen eines aus der Dialysierflüssigkeits - Kontamination herleitbaren Referenz - Werts für einen Systembetriebszustand (beispielsweise ein Blutfluss von 50 ml/min) bei dem bekannter Maßen keine Shunt - Rezirkulation vorherrscht,
- Hochfahren des Systems auf einen gewünschten (Ziel - ) Systembetriebszustand (beispielsweise ein Blutfluss von 300 ml/min) und Bestimmen eines aus der Dialysierflüssigkeits - Kontamination herleitbaren aktuellen/realen Werts für diesen Systembetriebszustand, bei dem eine Shunt - Rezirkulation angenommen wird,
- Bestimmen eines aus der Dialysierflüssigkeits - Kontamination herleitbaren idealen Werts für diesen Systembetriebszustand, bei dem idealer Weise keine Shunt - Rezirkulation vorliegt und
- Definieren der tatsächlichen Shunt - Rezirkulation für diesen

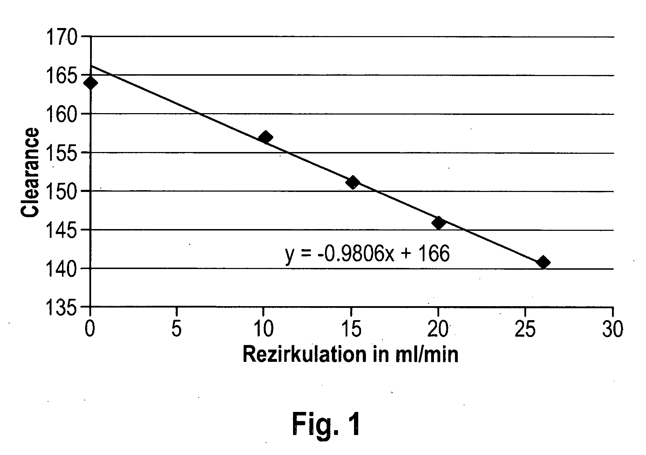

Systembetriebszustand direkt oder indirekt aus dem aktuellen und idealen Wert. Des Weiteren erkennt man insbesondere in der anliegenden Fig. 1 eine Gerade, die sich ergibt, wenn man verschiedene Rezirkulationsgrade erzeugt und den Einfluss auf die Reinigungsleistung des aktuell verwendeten Filters (Dialysators) gemäß der vorstehenden Verfahrensschritte misst/bestimmt. Man erkennt, dass die Steigung b dieser Geraden quasi (-1) (also negativ) ist, was bedeutet, dass die Clearanceänderung direkt in eine Rezirkulation Rez umgerechnet werden kann.

Aus dieser Überlegung ergibt sich die Berechnung der Shuntrezirkulation nach

$$Rez = K(soll) - K(gemessen) \qquad [in\ ml/min]$$

wobei

- Rez die Rezirkulation ist,
- K(gemessen) der beim aktuell eingestellten Blutfluss ermittelte Clearancewert ist und
- K(soll) der hinterlegte Wert für den speziellen Filter (Dialysator) entsprechend dem eingestellten Blutfluss sowie bei angenommener Komplettreinigung ist.

Sollte die Geradensteigung (Faktor b) ungleich 1 bzw. (- 1) (im Falle einer negativen Steigung) sein, ist sie als Faktor in der Umrechnung der Clearanceabweichung $\Delta K$ in den Rezirkulationswert Rez mit zu berücksichtigen. In diesem Fall ergibt sich:

$$Rez\ (\%) = [K(soll) - K(gemessen)] / (Faktor\ b) \qquad [in\ \%]$$

Es ist auch möglich, sequentielle Phasen (reine Ultrafiltration - auch als UF bezeichnet) für die Umsetzung zu verwenden. Die Berechnung erfolgt hier genauso wie vorstehend beschrieben, allerdings besteht keine Notwendigkeit mehr, den Filter zu kennen, da im UF - Modus urämische Toxine in ihrer Blutplasmakonzentration durch reine Konvektion auf die Dialysierflüssigkeitsseite geschwemmt werden. In anderen Worten ausgedrückt, da der Siebkoeffizient der Fasermembran eines Dialysators für kleinmolekulare Substanzen im allgemeinen bekannt ist, besteht bei diesem Modus keine Notwendigkeit der konkreten Filterkenntnis. (Siebkoeffizient ist hierbei wie folgt definiert: Man lässt Flüssigkeit über die Dialysatormembran strömen, hat hierbei jedoch keinen Dialysierflüssigkeitsfluss und bestimmt nun das Verhältnis aus den Konzentrationen einer Markersubstanz im Filtrat zur Eingangsflüssigkeit).
In dem UF - Modus genügt es somit, einen Messwert der Absorbanz bei dem gewählten (gewünschten) Behandlungsblutfluss von vorzugsweise 300 ml/min ins Verhältnis zum Absorbanzmesswert ohne Shuntrezirkulation (Referenzmesswert bei vorzugsweise 50 ml/min) zu setzen, wie dies vorstehend bereits ausgeführt wurde. Es gilt dann die vorstehend bereits genannte Formel:

$$Rez(\%) = (1 - (A(300) / A(50)) * 100 \qquad [allgemeine\ Formel]$$

Ein Hochrechnen des (Referenz-) Absorbanzwerts bei 50 ml/min Blutfluss auf den Behandlungsblutfluss (vorzugsweise 300 ml/min) ist nicht notwendig, weil nicht der Blutfluss entscheidend ist, sondern rein der eingestellte transmembrane (konvektive) Fluss.

Die Erfindung wird nachstehend anhand eines bevorzugten Ausführungsbeispiels unter Bezugnahme auf die begleitenden Figuren näher erläutert.

Fig 1 zeigt beispielhaft ein Clearance - Rezirkulations - Diagramm (zur Umrechnung eines Clearancewerts in einen Rezirkulationswert) für einen Dialysator bestimmten Typs,

Fig. 2 zeigt den prinzipiellen Aufbau eines Dialysators mit einer erfindungsgemäßen Messvorrichtung am Dialysatorausgang,

Fig. 3 zeigt einen Graph bezüglich der Intensität I (eines UV-Messsignals) bei unterschiedlichen System - Betriebszuständen und

Fig. 4 zeigt in Gegenüberstellung die grundsätzlichen Schritte des erfindungsgemäßen Steuerungsverfahrens eines Blutreinigungssystems im Hämofiltrationsmodus und im Ultrafiltrationsmodus.

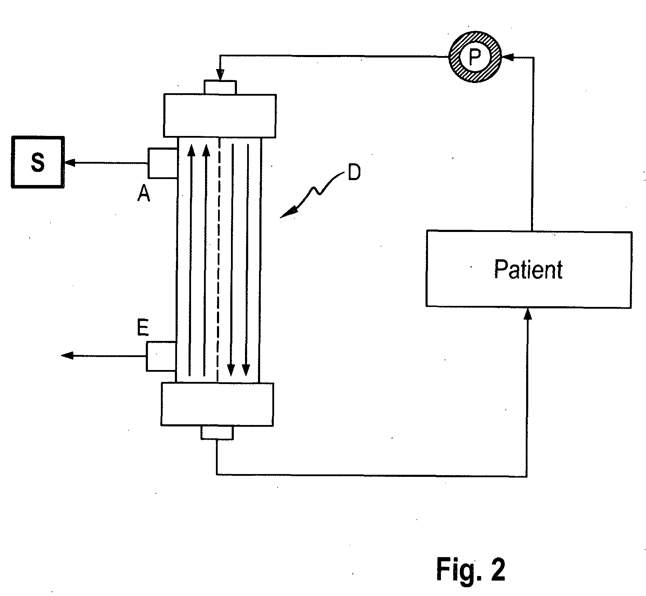

In der Fig. 2 ist dargestellt, wie über die Pumpe P Blut durch einen Dialysator D vorbestimmten Typs zirkuliert wird und anschließend zurück in den Patienten gegeben wird.

Innerhalb des Dialysators D wird vorzugsweise im Gegenstromprinzip eine Reinigungslösung (Dialysierflüssigkeit) in einen Dialysatoreingang E eingeleitet und dann um die Hohlfasern des Dialysators D bekannten Aufbaus gespült. Auf diese Weise werden bekannter Maßen urämische Substanzen aus dem Blutkreislauf entfernt und mit der Reinigungslösung (Dialysierflüssigkeit) abtransportiert. Im Ablauf A für die (verbrauchte) Reinigungslösung befindet sich ein Sensor (UV - Messeinrichtung) S, der ein Signal (nach dem Prinzip der UV - Spektrometrie) an einen nicht weiter gezeigten Rechner generiert, das mit einem Konzentrations - Größenwert (Absorbanzwert) bezüglich entfernter Substanzen (urämische Toxine) korreliert. Der korrelierende Konzentrations - Größenwert wird dann durch den Rechner bzw. durch eine Vergleichereinheit des Rechners mit einem theoretischen Konzentrations - Größenwert für diesen spezifischen Dialysator und unter theoretisch angenommen optimalen Bedingungen (ohne Rezirkualtion) verglichen, worauf basierend auf einer ermittelten Abweichung auf einen zugehörigen Rezirkulationsgrad beispielsweise unter Verwendung eines Diagramms gemäß der Fig. 1 rückgeschlossen wird.

Diese Vorrichtung kann somit beispelhaft im Fall einer Hämodialyse gemäß der Fig. 4 wie folgt konkret betrieben werden:

a. Zu Beginn einer Behandlung wird für ca. 2 min ein Blutfluss von 50 ml/min (Komplettreinigung und fehlende Rezirkulation) gefahren und die Referenz - Absorbanz der abfließenden Reinigungslösung bestimmt, beispielsweise A(50) = 0,458.

b. Nun wird der Blutfluss auf den Behandlungsfluss BF= 300 ml/min hoch geregelt und die Ist - Absorbanz erneut bestimmt, beispielsweise A(300) = 1,383.

c. Dann wird der theoretisch zu erwartende Soll - Wert für die Absorbanz A(300,theo) unter der Annahme von Komplettreinigung ohne Rezirkulation ermittelt, beispielsweise A(300,theo) = 2,748.

d. Schließlich wird die Clearance über das Verhältnisses aus gemessener Ist - Absorbanz A(300) und theoretisch erwarteter Soll - Absorbanz unter der Annahme von Komplettreinigung ohne Rezirkulation A(300,theo) berechnet.

$$K = [A(300) / A(300,theo)] * 300 \text{ ml/min} = 151 \text{ ml/min}.$$

e. Abschließend wird die Rezirkulation über den für diesen Filter ermittelten Zusammenhang bestimmt, beispielsweise aus

$$Rez \text{ (ml/min)} = [K(soll) - K(gemessen)] / b = (166\text{-}151) \text{ ml/min} / 1 = 15 \text{ ml/min}.$$

Somit beträgt die Rezirkulation für dieses Beispiel 15 ml/min des Blutflusses. Die Einheit speziell in diesem Beispiel ist ml/min (Blutfluss). Grundsätzlich könnte die Einheit der Rezirkulation aber auch anders angegeben werden - beispielsweise in %.

Nach der Bestimmung der Rezirkulation im Shunt ist es ferner möglich, den theoretischen Shuntfluss zu bestimmen, sofern die Rezirkulation ungleich, d.h. größer 0% ist. Hierfür existieren bereits Berechnungsformeln nach Mercadel, die in diesem Fall angewendet werden können. Insbesondere gilt:

$$Qa = (Qb - UF) + (1 - R) / R$$

mit

Qa: theoretischer Shuntfluss
Qb: Blutfluss
UF: Ultrafiltrationswert
R: Rezirkulationsgrad

Sofern die vorstehend genannte Bestimmung des Shuntflusses auf diese Weise nicht möglich sein sollte, etwa weil die Rezirkulation 0% beträgt, kann durch ein Vertauschen des venösen und arteriellen Zugangs am Shunt das Blutsystem quasi im inversen Betrieb gefahren werden. Dazu existieren im Stand der Technik bereits verschiedene mechanische Vorrichtungen zum Vereinfachen eines solchen Umschaltvorgangs vom Normalbetrieb in den Inversbetrieb.

Sofern dann das System in dem inversen Modus betrieben wird, tritt eine dadurch erzwungene Rezirkulation auf. Auf diese Weise wird es ermöglicht, durch die Bestimmung der Clearance für beide Fälle gemäß vorstehender Beschreibung mit der Formel zu Mercadel den theoretischen Shuntfluss zu bestimmen. Hierbei gilt:

$$Qa = (K1 * K2) / (K1 - K2)$$

wobei

K1: Clearance im Normalbetrieb
K2: Clearance im Inversbetrieb nach dem Umschalten

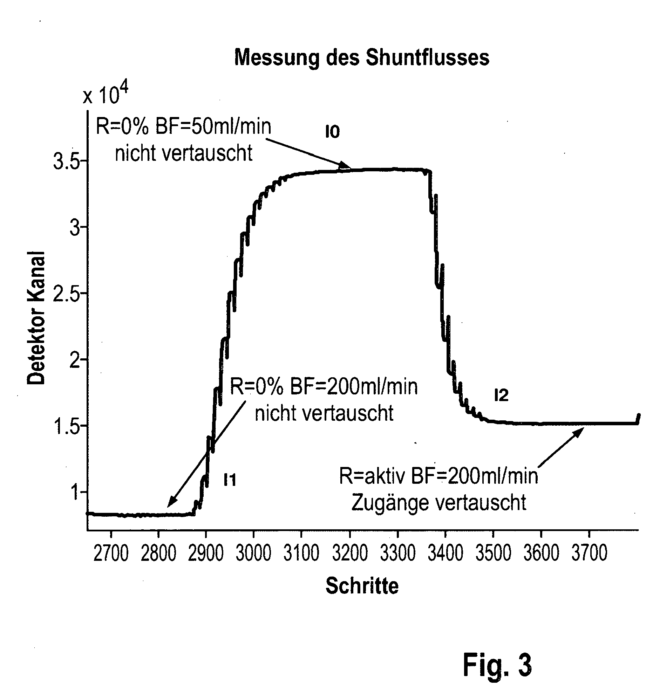

Die Fig. 3 zeigt hierfür ein Diagramm, in welchem die Intensität für einen Behandlungsblutfluss von 200 ml/min bei einer Rezirkulation von 0%, anschließendem Absenken des Blutflusses auf 50 ml/min bei einer Rezirkulation von weiterhin 0% und abschließendem Umschalten in den Inversbetrieb bei gleichzeitigem Anheben des Blutflusses erneut auf 200 ml/min dargestellt ist.

Aus den graphisch dargestellten Intensitätswerten I1, I0 und I2 für die vorstehenden Blutflusskenngrößen: 200 ml/min (Normalbetrieb), 50 ml/min (Normalbetrieb), 200 ml/min (Inversbetrieb) lassen sich die Clearance K1 und K2 vor und nach dem Umschalten errechnen. Hierfür gilt:

$$K1 = I1 * Qb1 / I0$$

und

$$K2 = I2 * Qb2 / I0$$

mit

Qb1: Bluttluss vor dem Umschalten (beispielsweise hier 200 ml/min)
Qb2: Blutfluss nach dem Umschalten (beispielsweise hier 200 ml/min)

Zusammenfassend betrifft die vorliegende Erfindung ein Verfahren und eine Vorrichtung zur Bestimmung einer Rezirkulation während einer Dialyse anhand der Dialysesystemantwort auf eine Systembetriebsgrößenänderung mit folgenden grundsätzlichen Verfahrensschritten:

- Bestimmen eines aus der Dialysierflüssigkeits - Kontamination herleitbaren Referenz - Werts für einen Systembetriebszustand (beispielsweise ein Blutfluss von 50 ml/min) bei dem bekannter Maßen keine Shunt - Rezirkulation vorherrscht,
- Hochfahren des Systems auf einen gewünschten (Soll - ) Systembetriebszustand (beispielsweise ein Blutfluss von 300 ml/min) und Bestimmen eines aus der Dialysierflüssigkeits - Kontamination herleitbaren aktuellen/realen Werts für diesen Systembetriebszustand, bei dem eine Shunt - Rezirkulation angenommen wird,

- Bestimmen eines aus der Dialysierflüssigkeits - Kontamination herleitbaren idealen Werts für diesen Systembetriebszustand, bei dem idealer Weise keine Shunt - Rezirkulation vorliegt und
- Definieren der tatsächlichen Shunt - Rezirkulation für diesen Systembetriebszustand direkt oder indirekt aus dem aktuellen und idealen Wert.

**Patentansprüche**

1. Steuerungsverfahren für ein Blutreinigungssystems, unter anderem bestehend aus mindestens einem Bluteinlass-/Auslasselement, vorzugsweise einem Shunt, und einer Blutreinigungsmaschine, an die das Bluteinlass-/Auslasselement angeschlossen oder anschließbar ist, wobei das Steuerungsverfahren dafür angepasst ist, zur Bestimmung einer Rezirkulation während einer extrakorporalen Blutbehandlung mittels des Blutreinigungssystems anhand der Systemantwort auf eine Systembetriebsgrößenänderung folgende Verfahrensschritte auszuführen:

   - Bestimmen eines aus der Dialysierflüssigkeitskontamination herleitbaren Referenzwerts für einen Systembetriebszustand bzw. eine Systembetriebsgröße, bei dem bekannter Maßen keine Rezirkulation vorherrscht oder die Rezirkulation vorbekannt ist,
   - Betreiben des Systems bei einem gewünschten Ziel-Systembetriebszustand bzw. einer gewünschten Ziel-Systembetriebsgröße, angepasst für eine Patientenbehandlung, und Bestimmen eines aus der Dialysierflüssigkeitskontamination herleitbaren aktuellen Werts für den Ziel-Systembetriebszustand bzw. die Ziel-Systembetriebsgröße,
   - Bestimmen eines aus der Dialysierflüssigkeitskontamination herleitbaren idealen Werts für den Ziel-Systembetriebszustand bzw. die Ziel-Systemsbetriebsgröße, bei dem idealer Weise keine Rezirkulation vorliegt, und
   - Berechnen der tatsächlichen Rezirkulation für den Ziel-Systembetriebszustand bzw. die Ziel-Systemsbetriebsgröße direkt oder indirekt aus dem aktuellen und dem idealen Wert.

2. Steuerungsverfahren für ein Blutreinigungssystem, insbesondere nach Anspruch 1, unter anderem bestehend aus mindestens einem Bluteinlass-/Auslass- Element, vorzugsweise einem Shunt, und einer Blutreinigungsmaschine, an die das mindestens eine Bluteinlass-/Auslass-Element angeschlossen oder anschließbar ist, wobei das Steuerungsverfahren dafür angepasst ist, zur Bestimmung einer Rezirkulation während einer extrakorporalen Blutbehandlung mittels des Blutreinigungssystems anhand der Systemantwort auf eine Systembetriebsgrößenänderung folgende Verfahrensschritte auszuführen:

   - Reinigungsflüssigkeitsseitiges Ermitteln einer sensorisch messbaren, die Konzentration von urämischen Toxinen aus dem extrakorporalen Kreislauf repräsentierenden Referenzkenngröße für eine vorbestimmte Referenz-Systembetriebsgröße, bei welcher der Zustand einer Komplettreinigung ohne Rezirkulation vorliegt, und Berechnen einer Sollkenngröße für eine Zielsystembetriebsgröße aus der Referenzkenngröße,
   - Einstellen der Ziel-Systembetriebsgröße und Ermitteln einer Ist -Kenngröße bezogen auf die Zielsystembetriebsgröße,
   - Bestimmen eines Ist-Vergleichswerts aus der Ist- und Referenzkenngröße oder aus der Ist- und Soll-Kenngröße bezogen auf die Ziel- Systembetriebsgröße und Ermitteln der absoluten oder relativen Differenz zwischen dem Ist-Vergleichswert und einem systemspezifischen Soll-Vergleichswert bezogen auf die Ziel-Systembetriebsgröße,
   - Umrechnen des Differenzwerts in einen hierzu korrelierenden Rezirkulationsgrad.

3. Steuerungsverfahren nach Anspruch 2, **dadurch gekennzeichnet, dass** die Systembetriebsgröße ein Blutfluss, vorzugsweise in ml/min, eine Förderrate eines Fördermittels, eine Umdrehungszahl oder ein Hub des Fördermittels oder dergleichen Volumenstrom repräsentierende Leistungsgröße ist, die Kenngröße ein Absorbanzwert oder dergleichen optischer oder elektromagnetischer Eigenschaftswert von urämischen Substanzen ist und der Vergleichswert eine Clearance vorzugsweise in ml/min ist.

4. Steuerungsverfahren nach Anspruch 3, **dadurch gekennzeichnet, dass** der Soll-Clearancewert als der Soll-Vergleichswert ein für den Zielblutfluss als Zielsystembetriebsgröße vorab ermittelter, Dialysator-spezifischer Soll-Clearancewert ist, bei welchem für den Zielblutfluss ein im Wesentlichen rezirkulationsfreier Zustand vorliegt.

5. Steuerungsverfahren nach Anspruch 3 oder 4, **dadurch gekennzeichnet, dass** zur Bereitstellung des Vergleichsergebnisses die Differenz zwischen dem Soll-Clearancewert und dem Ist-Clearancewert gebildet wird, wobei der Ist-Clearancewert aus dem Verhältnis des gemessenen Ist-Absorbanzwerts zu dem aus der Referenz-Absorbanz berechneten Soll-Absorbanzwert für den Zielblutfluss errechnet ist.

6. Steuerungsverfahren nach einem der Ansprüche 3 bis 5, **dadurch gekennzeichnet, dass** als Referenzblutfluss 50 ml/min und als Zielblutfluss 300 ml/min gewählt wird.

7. Steuerungsverfahren nach einem der vorstehenden Ansprüche 3 bis 6, **dadurch gekennzeichnet, dass** bei einem errechneten Rezirkulationsgrad von ungleich 0% ein theoretischer Shuntfluss aus den bekannten Größen

    a. Zielblutfluss
    b. Ultrafiltrationswert
    c. Rezirkulationsgrad

berechnet wird.

8. Steuerungsverfahren nach einem der vorstehenden Ansprüche 3 bis 6, **dadurch gekennzeichnet, dass** bei einem Rezirkulationsgrad von 0% ein theoretischer Shuntfluss mit den folgenden Schritten bestimmbar ist:

    a. Ermitteln der Ist-Clearance für eine Ist-Strömungsrichtung des Blutflusses im Shunt,
    b. Ermitteln einer Referenzclearance bei einer zur Ist-Strömungsrichtung inversen Strömungsrichtung des gleichen Blutflusses im Shunt,
    c. Bestimmen des theoretischen Shuntflusses aus dem Produkt aus der Ist- und Referenzclearance dividiert durch die Differenz zwischen der Ist- und der Referenzclearance.

9. Steuerungsverfahren für ein Blutreinigungssystem, insbesondere nach Anspruch 1, unter anderem bestehend aus mindestens einem Bluteinlass-/Auslass- Element, vorzugsweise einem Shunt, und einer Blutreinigungsmaschine, an die das mindestens eine Bluteinlass-/Auslass-Element angeschlossen oder anschließbar ist, wobei das Steuerungsverfahren dafür angepasst ist, zur Bestimmung einer Rezirkulation während einer extrakorporalen Blutbehandlung mittels des Blutreinigungssystems anhand der Systemantwort auf eine Systembetriebsgrößenänderung folgende Verfahrensschritte auszuführen:

    a. Messung einer die Dialysierflüssigkeitskontamination darstellenden Referenzkenngröße, vorzugsweise der Referenzabsorbanz (A(50)), bei einem Referenzblutfluss von vorzugsweise 50 ml/min unter der Voraussetzung fehlender Rezirkulation,
    b. Messung einer die Dialysierflüssigkeitskontamination darstellenden Ist-Kenngröße, vorzugsweise der Ist-Absorbanz (A(BBF)), bei dem gewünschten Soll-Behandlungsfluss (A(BBF)), vorzugsweise bei 300ml/min als Systemreaktion,
    c. Gleichsetzen der gemessenen Referenzkenngröße mit einer Soll-Kenngröße, vorzugsweise dem theoretischen Soll-Absorbanzwert (A(BBF,theo)), für eine angenommene, theoretische Rezirkulationsfreiheit beim gewählten Behandlungsfluss (BBF) als identischer Wert: A(50) = A(BBF,theo),
    d. aus dem Verhältnis der gemessenen Ist-Kenngröße, vorzugsweise des Ist-Absorbanzwerts (A(BBF)), zu der Soll-Kenngröße, vorzugsweise dem Soll-Absorbanzwert (A(BBF, theo)), der dem Referenzabsorbanzwert A(50) entspricht, unmittelbares Ermitteln der Rezirkulation (Rez) aus:

$$Rez \text{ (in \%)} = (1 - (A(BBF) / A(50))) * 100$$

oder

$$Rez \text{ (in ml/min)} = (1 - (A(BBF) / A(50))) * BFF.$$

10. Vorrichtung zur extrakorporalen Blutbehandlung , **dadurch gekennzeichnet, dass** die genannte Vorrichtung eine Steuereinrichtung aufweist, die so angepasst ist, dass sie das Verfahren gemäß der Ansprüche 1 bis 9 ausführt und wobei einem Dialysator nur ein einziger Sensor dialysierflüssigkeitsseitig, vorzugsweise unmittelbar in der Dialysierflüssigkeitsstromrichtung, nachgeordnet ist, der zur Messung der Referenzkenngröße und Ist-Kenngröße vorgesehen ist.

**11.** Vorrichtung nach Anspruch 10, **dadurch gekennzeichnet, dass** der Sensor für eine Absorbanzmessung angepasst ist.

**12.** Vorrichtung nach einem der Ansprüche 10 bis 11, **gekennzeichnet durch** einen Rechner mit einer Speichereinheit, in der eine Anzahl von Soll-Vergleichswerten, vorzugsweise Soll-Clearancewerten bezogen auf ausgewählte Ziel-Systembetriebsgrößen, vorzugsweise Ziel-Blutströme abgelegt sind, bei denen jeweils eine Komplettreinigung vorliegt.

**13.** Vorrichtung nach einem der Ansprüche 10 bis 12, **gekennzeichnet durch** zumindest eine Blutpumpe, die dafür angepasst ist, den Blutfluss ausgehend von dem Referenzblutflusswert auf den Ziel-Behandlungsblutflusswert sprungartig zu verändern.

**Claims**

**1.** A control method for a blood purification system, inter alia consisting of at least one blood inlet/outlet element, preferably a shunt, and a blood purification apparatus to which the blood inlet/outlet element is connected or can be connected, the control method being adapted, for determining a recirculation during an extracorporeal treatment of the blood by means of the blood purification system and on the basis of the system response to an alteration of a system-related operating value, to carry out the following method steps:

- determining a reference value, which can be derived from the contamination of a dialysis liquid, for a system-related operational state or a system-related operating value for which - as is known - there is no recirculation or the recirculation is known in advance,
- operating the system at a desired system-related target operational state or at a desired system-related target operational value, being adapted for a treatment of a patient, and determining a current value, which can be derived from the contamination of the dialysis liquid, for the system-related target operational state or the system-related target operational value,
- determining an ideal value, which can be derived from the contamination of the dialysis liquid, for the system-related target operational state or the system-related target operational value, for which there is no recirculation in the ideal case, and
- calculating the true recirculation for the system-related target operational state or the system-related target operational value directly or indirectly from the current value and the ideal value.

**2.** A control method for a blood purification system, in particular according to claim 1, inter alia consisting of at least one blood inlet/outlet element, preferably a shunt, and a blood purification apparatus to which the at least one blood inlet/outlet element is connected or can be connected, the control method being adapted, for determining a recirculation during an extracorporeal treatment of the blood by means of the blood purification system and on the basis of the system response to an alteration of the system-related operating value, to carry out the following method steps:

- establishing, at the side of the purification liquid, a reference parameter which can be measured by sensor technology and represents the concentration of uremic toxins from the extracorporeal circulation for a predetermined system-related reference operating value at which there exists a state of complete purification without any recirculation, and calculating a target parameter for a system-related target operational value from the reference parameter,
- presetting the system-related target operational value and establishing an actual parameter related to the system-related target operational value,
- determining an actual comparative value from the actual parameter and the reference parameter or from the actual parameter and the target parameter related to the system-related target operational value, and establishing the absolute or relative difference between the actual comparative value and a system-specific target comparative value related to the system-related target operational value,
- converting the differential value into a recirculation degree correlating thereto.

**3.** The control method according to claim 2, **characterized in that** the system-related operating value is a blood flow, preferably in ml/min, a flow rate of a conveyor means, a rotational speed or a stroke of the conveyor means or a similar performance variable representing a volume flow, the characteristic parameter is an absorbance value or a similar optical or electromagnetic property value of uremic substances, and the comparative value is a clearance preferably in ml/min.

4. The control method according to claim 3, **characterized in that** the target clearance value as the target comparative value is a dialyzer-specific target clearance value previously established for the target blood flow as a system-related target operational value, at which a state with essentially no recirculation exists for the target blood flow.

5. The control method according to claim 3 or 4, **characterized in that** for providing a comparison result, the difference between the target clearance value and the actual clearance value is calculated, the actual clearance value being calculated from the ratio between the measured actual absorbance value and the target absorbance value for the target blood flow calculated from the reference absorbance.

6. The control method according to any of claims 3 to 5, **characterized in that** the reference blood flow is selected to be 50 ml/min and the target blood flow is selected to be 300 ml/min.

7. The control method according to any of preceding claims 3 to 6, **characterized in that** in case a calculated recirculation degree is unequal to 0%, a theoretical shunt flow is calculated from the known variables

   a. target blood flow
   b. ultrafiltration value
   c. recirculation degree.

8. The control method according to any of preceding claims 3 to 6, **characterized in that** in case a recirculation degree amounts to 0%, a theoretical shunt flow can be determined by means of the following steps:

   a. establishing the actual clearance for an actual flow direction of the blood flow in the shunt,
   b. establishing a reference clearance with a flow direction which is inverse to the actual flow direction, of the same blood flow in the shunt,
   c. determining the theoretical shunt flow from the product of the actual clearance and the reference clearance divided by the difference between the actual clearance and the reference clearance.

9. A control method for a blood purification system, in particular according to claim 1, inter alia consisting of at least one blood inlet/outlet element, preferably a shunt, and a blood purification apparatus to which the at least one blood inlet/outlet element is connected or can be connected, the control method being adapted, for determining a recirculation during an extracorporeal treatment of the blood by means of the blood purification system and on the basis of the system response to an alteration of the system-related operating value, to carry out the following method steps:

   a. measuring a reference parameter representing the contamination of the dialysis liquid, preferably the reference absorbance (A(50)), with a reference blood flow of preferably 50 ml/min and with the prerequisite that there is no recirculation,
   b. measuring an actual parameter representing the contamination of the dialysis liquid, preferably the actual absorbance (A(BBF)), with the desired target treatment flow (A(BBF), preferably at 300ml/min as a system reaction,
   c. equating the measured reference parameter and a target parameter, preferably the theoretical target absorbance value (A(BBF,theo)), for an assumed, theoretical absence of recirculation for the selected treatment flow (BBF) as an identical value: A(50) = A(BBF,theo),
   d. directly establishing the recirculation (Rec) from the ratio between the measured actual parameter, preferably the actual absorbance value (A(BBF)), and the target parameter, preferably the target absorbance value (A(BBF, theo)) corresponding to the reference absorbance value A(50), by means of the following:

$$Rec \text{ (in \%)} = (1 - (A(BBF) / A(50)) * 100$$

   or

$$Rec \text{ (in ml/min)} = (1 - (A(BBF) / A(50)) * BFF.$$

10. A device for the extracorporeal treatment of blood **characterized in that** the device includes control means adapted so as to carry out the method according to any of claims 1 to 9 and wherein only a single sensor is arranged downstream a dialyzer on the side of the dialysis liquid and preferably directly in the flow direction of the dialysis liquid, said sensor being provided for measuring the reference parameter and the actual parameter.

11. The device according to claim 10, **characterized in that** the sensor is adapted for measuring an absorbance.

12. The device according to any of claims 10 to 11, **characterized by** a computer comprising a memory unit storing a number of target comparative values, preferably target clearance values related to selected system-related target operational values, preferably target blood flows, for which complete purification is available.

13. The device according to any of claims 10 to 12, **characterized by** at least one blood pump which is adapted for changing the blood flow starting from the reference blood flow value to the treatment blood flow target value in an abrupt manner.

## Revendications

1. Procédé de commande destiné à un système de purification du sang, constitué entre autres au moins d'un élément d'entrée/de sortie du sang, de préférence d'un shunt, et d'une machine de purification du sang, à laquelle l'élément d'entrée/de sortie du sang est ou peut être raccordé, le procédé de commande étant adapté pour exécuter, afin de définir une recirculation, au cours d'un traitement du sang extracorporel au moyen du système de purification du sang, à l'aide de la réponse du système à une modification de paramètre de fonctionnement de système, les étapes de procédé qui suivent :

   - la définition d'une valeur de référence pouvant être déduite de la contamination de liquide de dialyse pour un état de fonctionnement de système ou un paramètre de fonctionnement de système, pour laquelle, comme on le sait, aucune circulation ne règne ou la recirculation est préalablement connue ;
   - le fonctionnement du système à un état de fonctionnement de système cible souhaité ou à un paramètre de fonctionnement de système cible souhaité, de manière adaptée à des soins de patient, et la détermination d'une valeur applicable pouvant être déduite de la contamination de liquide de dialyse pour l'état de fonctionnement de système cible ou pour le paramètre de fonctionnement de système cible ;
   - la définition d'une valeur idéale pouvant être déduite de la contamination de liquide de dialyse pour l'état de fonctionnement de système cible et pour le paramètre de fonctionnement de système cible, pour laquelle idéalement aucune recirculation n'est de mise ; et
   - le calcul de la recirculation réelle pour l'état de fonctionnement de système cible ou le paramètre de fonctionnement de système cible directement ou indirectement à partir de la valeur applicable ou de la valeur idéale.

2. Procédé de commande pour un système de purification de sang, en particulier selon la revendication 1, constitué entre autres au moins d'un élément d'entrée/de sortie du sang, de préférence d'un shunt, et d'une machine de purification de sang, à laquelle le ou les éléments d'entrée/de sortie du sang sont ou peuvent être raccordés, le procédé de commande étant adapté pour exécuter, afin de définir une recirculation, au cours d'un traitement du sang extracorporel au moyen du système de purification de sang, à l'aide de la réponse du système à une modification de paramètre de fonctionnement de système, les étapes de procédé qui suivent :

   - la détermination, côté liquide de purification, d'un paramètre caractéristique de référence pouvant être mesuré à l'aide de capteurs, représentant la concentration de toxines urémiques issues du circuit extracorporel, pour un paramètre de fonctionnement de système de référence préalablement défini, pour lequel l'état d'une purification complète sans recirculation est de mise, et le calcul d'un paramètre caractéristique de consigne pour un paramètre de fonctionnement de système cible à partir du paramètre caractéristique de référence ;
   - le réglage du paramètre de fonctionnement de système cible et la détermination d'un paramètre caractéristique réel par rapport au paramètre de fonctionnement de système cible ;
   - la définition d'une valeur de comparaison réelle à partir du paramètre caractéristique réel et de référence ou à partir du paramètre caractéristique réel et de consigne par rapport au paramètre de fonctionnement de système cible, et la détermination de la différence absolue ou relative entre la valeur de comparaison réelle et une valeur de comparaison de consigne propre au système par rapport au paramètre de fonctionnement de système cible ;
   - la conversion d'une valeur de référence en un degré de recirculation en corrélation.

3. Procédé de commande selon la revendication 2, **caractérisé en ce que** le paramètre de fonctionnement de système est un flux de sang, de préférence exprimé en ml/min, un taux de refoulement d'un produit de refoulement, une vitesse de rotation complète ou une course du produit de refoulement ou un paramètre de puissance représentant un flux volumique similaire, **en ce que** le paramètre caractéristique est une valeur d'absorbance ou une valeur de propriété similaire optique ou électromagnétique de substances urémiques, ou **en ce que** la valeur de comparaison est une clairance de préférence exprimée en ml/min.

4. Procédé de commande selon la revendication 3, **caractérisé en ce que** la valeur de clairance de consigne en tant que valeur de comparaison de consigne est une valeur de clairance de consigne déterminée au préalable pour le flux de sang cible en tant que paramètre de fonctionnement cible, propre au dialyseur, pour laquelle un état essentiellement sans recirculation est de mise pour le flux de sang cible.

5. Procédé de commande selon la revendication 3 ou 4, **caractérisé en ce que** la différence entre la valeur de clairance de consigne et la valeur de clairance réelle est formée pour fournir le résultat de comparaison, la valeur de clairance réelle étant calculée à partir du rapport entre la valeur d'absorbance réelle mesurée et la valeur d'absorbance de consigne calculée à partir de l'absorbance de référence.

6. Procédé de commande selon l'une quelconque des revendications 3 à 5, **caractérisé en ce qu'**on choisit en tant que flux de sang de référence 50 ml/min ou en tant que flux de sang cible 300 ml/min.

7. Procédé de commande selon l'une quelconque des revendications précédentes 3 à 6, **caractérisé en ce qu'**un flux de shunt théorique est calculé à partir des paramètres connus

   a. flux de sang cible ;
   b. valeur d'ultrafiltration ;
   c. degré de recirculation

pour un degré de recirculation calculé différent de 0 %.

8. Procédé de commande selon l'une quelconque des revendications précédentes 3 à 6, **caractérisé en ce qu'**un flux de shunt théorique peut être déterminé, pour un degré de recirculation de 0 %, à l'aide des étapes qui suivent :

   a. la détermination de la clairance réelle pour une direction d'écoulement réelle du flux de sang dans le shunt ;
   b. la détermination de la clairance de référence pour une direction d'écoulement inverse par rapport à la direction d'écoulement réelle du flux de sang similaire dans le shunt ;
   c. la définition du flux de shunt théorique à partir du produit de la clairance réelle et de la clairance de référence, divisé par la différence entre la clairance réelle et la clairance de référence.

9. Procédé de commande pour un système de purification de sang, en particulier selon la revendication 1, constitué entre autres au moins d'un élément d'entrée/de sortie du sang, de préférence d'un shunt, et d'une machine de purification de sang, à laquelle l'élément ou les éléments d'entrée/de sortie du sang sont ou peuvent être raccordés, le procédé de commande étant adapté pour exécuter, afin de définir une recirculation, au cours d'un traitement du sang extracorporel au moyen du système de purification de sang, à l'aide de la réponse du système à une modification de paramètre de fonctionnement de système, les étapes de procédé qui suivent :

   a. la mesure d'un paramètre caractéristique de référence représentant la contamination de liquide de dialyse, de préférence de l'absorbance de référence (A(50)), pour un flux de sang de référence de préférence de 50 ml/min sous réserve d'une absence de recirculation ;
   b. la mesure d'un paramètre caractéristique réel représentant la contamination de liquide de dialyse, de préférence de l'absorbance réelle (A(BBF)) pour le flux de traitement de consigne souhaité (A(BBF)), de préférence à 300 ml/min en tant que réaction du système ;
   c. la mise en équation du paramètre caractéristique de référence mesuré avec un paramètre caractéristique de consigne, de préférence la valeur d'absorbance de consigne théorique (A(BBF, théo), pour une liberté de recirculation théorique admise pour un flux de traitement (BBF) choisi en tant que valeur identique : A(50) = A(BBF, théo) ;
   d. la détermination directe de la recirculation Rec à partir du rapport du paramètre caractéristique réel mesuré, de préférence de la valeur d'absorbance réelle (A(BBF)), par rapport au paramètre caractéristique de consigne, de préférence la valeur d'absorbance de consigne (A(BBF, théo)), qui correspond à la valeur d'absorbance de référence A(50), à partir de :

$$Rec \text{ (en \%)} = (1 - (A(BBF) / A(50)) * 100$$

ou

$$Rec \text{ (en ml/min)} = (1 - (A(BBF)) / A(50)) * BFF.$$

**10.** Dispositif servant au traitement du sang extracorporel, **caractérisé en ce que** ledit dispositif présente un système de commande, qui est adapté de telle sorte qu'il exécute le procédé selon les revendications 1 à 9, un seul capteur étant disposé en aval d'un dialyseur, côté liquide de dialyse, de préférence directement dans la direction d'écoulement de liquide de dialyse, lequel capteur est prévu afin de mesurer le paramètre caractéristique de référence et le paramètre caractéristique réel.

**11.** Dispositif selon la revendication 10, **caractérisé en ce que** le capteur est adapté pour une mesure d'absorbance.

**12.** Dispositif selon l'une quelconque des revendications 10 à 11, **caractérisé par** un calculateur comprenant une unité de stockage, dans laquelle un nombre donné de valeurs de comparaison de consigne, de préférence de valeurs de clairance de consigne par rapport à des paramètres de fonctionnement de système cibles, de préférence des flux de sang cibles est stocké, pour lesquels une purification complète est respectivement de mise.

**13.** Dispositif selon l'une quelconque des revendications 10 à 12, **caractérisé par** au moins une pompe à sang, qui est adaptée pour modifier de manière exponentielle le flux de sang en partant de la valeur de flux de sang de référence pour l'établir à la valeur de flux de sang de traitement cible.

Fig. 1

**Fig. 2**

Messung des Shuntflusses

Fig. 3

Blutfluss 50 ml/min [A(50)]

HD - Modus

reine Filtration

Umrechnen der Absorbanz in
den Wert, der bei Komplettreinigung
unter Behandlungsblutfluss
gemessen würde
(theoretische Wert ohne Rez.)

A(BBF, theo) = A(50) * (BBF/50)

Bei Behandlungsblutfluss
ohne Rezirkulation ergäbe
sich die gleiche Absorbanz

A(BBF, theo) = A(50)

K(BBF)=
A(BBF, ist) / A (BBF/theo) * BBF

K(BBF)=
A(BBF, ist) / A (BBF/theo) * BBF

Rez(%)=
(1-(K(BBF)/K(BBF,theo))*100

Rez(ml/min)=
(1-(K(BBF)/K(BBF,theo))*100

Rez(%)=
(1-(A(BBF)/A(50))*100

Rez(ml/min)=
(1-A(BBF)/A(50))*BBF

## Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0900094 A
- WO 2011026647 A1
- WO 2012062257 A1
- US 7815852 B
- US 5685989 A
- EP 1083948 A